# EUROPEAN PATENT SPECIFICATION

(11) **EP 0 805 692 B1**
(45) Date of publication and mention of the grant of the patent: **27.06.2001**
(21) Application number: 96901064.4
(22) Date of filing: 29.01.1996
(51) Int. Cl.: A61L 26/00

(54) **ENHANCEMENT OF ACTIVATION FOR "BIOLOGICAL" TISSUE ADHESIVES, BONDING AGENTS AND SEALANTS USING "COLOUR CHANGE" CHROMOPHORES**
VERSTÄRKUNG DER AKTIVIERUNG VON BIOLOGISCHEN GEWEBEKLEBSTOFFEN, BINDUNGSMITTELN UND VERSIEGLERN UNTER VERWENDUNG DER FARBENÄNDERUNG VON CHROMOPHOREN
AMELIORATION DE L'ACTIVATION D'ADHESIFS "BIOLOGIQUES" POUR TISSUS, AGENTS LIANTS ET AGENTS DE SCELLEMENT FAISANT APPEL A DES CHROMOPHORES A "COULEUR CHANGEANTE"

(30) Priority: 27.01.1995 GB 9501579
(43) Date of publication of application: 12.11.1997
(73) Proprietor: Tissuemed Limited, Leeds LS14 6UF (GB)
(72) Inventor: WILKINSON, Francis, Loughborough, Leicestershire LE11 3JR (GB); MANDLEY, David, John, Sheffield, South Yorkshire S31 0XX (GB)
(74) Representative: Perry, Robert Edward
(86) International application number: PCT/GB96/00181
(87) International publication number: WO 96/22797

(56) References cited:
- WO-A-92/02238
- THE JOURNAL OF THORACIC AND CARDIOVASCULAR SURGERY, vol. 103, no. 4, April 1992, pages 781-783, XP000572402 J.S. AUTERI ET AL.: "LASER ACTIVATION OF TISSUE SEALANT IN HAND-SEWN CANINE ESOPHAGEAL CLOSURE"
- LASERS IN SURGERY AND MEDICINE, vol. 13, no. 5, 1993, US, pages 577-580, XP000397035 D.P. POPPAS ET AL.: "PREPARATION OF HUMAN ALBUMIN SOLDER FOR LASER TISSUE WELDING"
- JOURNAL OF CLINICAL LASER MEDICINE & SURGERY, vol. 11, no. 3, June 1993, pages 123-126, XP002004722 M.C. OZ ET AL.: "LASER-ASSISTED FIBRINOGEN BONDING OF UMBILICAL VEIN GRAFTS"
- ARCHIVES OF SURGERY, vol. 125, no. 11, November 1990, pages 1452-1454, XP000572196 N. MOAZAMI ET AL.: "REINFORCEMENT OF COLONIC ANASTOMOSES WITH A LASER AND DYE-ENHANCED FIBRINOGEN"
- PROCEEDINGS OF LASERS IN OTOLARYNGOLOGY, DERMATOLOGY, AND TISSUE WELDING, vol. 1876, January 1993, US, pages 175-179, XP002004723 M.M. JUDY ET AL.: "HEAT FREE PHOTOCHEMICAL TISSUE WELDING WITH 1,8-NAPHTHALIMIDE DYES USING VISIBLE (420 NM) LIGHT"

## Description

This invention relates to tissue adhesives.

Tissue adhesives may be used instead of suturing and stapling, for example in surgical procedures, for repair or the creation of anastomoses. They may also be used as dressings, haemostats or sealants.

The application of a suitable biocompatible adhesive offers many advantages to patient and surgeon alike. It avoids penetration of tissues by needles, as well as immediately sealing the treated tissue. It can prevent or minimise foreign body reaction and scarring. A suitable substance could be quicker to apply and its use may be more accurate than standard suturing, particularly where small vessels or structures are concerned. There is also the potential advantage that, if the energy required to set the adhesive is sufficiently low, analgesia may not be required.

The ideal tissue adhesive would be non-toxic and biocompatibles, strong and supple. It would not swell unduly after setting, would be user-friendly to apply and would be easy to sterilise and prepare.

WO 92/02238 discloses adhesive compositions that may be used to bond separated tissues together or to coat tissues or prosthetic material, to enhance strength and water-tightness, upon the application of laser radiation. Typical Examples show a composition comprising albumin and a green dye. Laser irradiation caused whitening.

The activation of a tissue adhesive including indocyanine green dye is also disclosed by Auteri *et al,* J. Thoracic Cardiovascular Surgery 103(4):781-3 (1992); Oz et *al,* J. Clin. Laser Med. Surg. 11(3):123-6 (1993); and Moazami et *al,* Arch. Surg. 125:1452-4 (1990). The first and last of these articles respectively report that the end point is indicated by "visible shrinking and desiccation of the sealant", and the "characteristic drying of the glue".

A major advantage of lasers is that laser energy can be applied precisely to selected areas, owing to the coherent nature and hence narrow beam feature of laser emission. Importantly the beam can be very easily end conveniently directed even to difficult access surgical sites by using optical fibre arrangements.

The use of endogenous or exogenous chromophores significantly enhances laser use. Incorporating suitable chromophores into the adhesive has several advantages. The chromophores, of which many have been quoted and used, selectively absorb the laser light energy and convert that energy to thermal energy where it has been applied. The chromophore is chosen for any specific wavelength. The heat generated then sets the adhesive. Thus chromophores enhance accuracy and distribute setting energy. Suitably chosen chromophores will also help reach setting temperatures quickly, thus reducing the time factor for laser application and importantly the overall energy requirements. On account of the selectivity of the chromophores for any given wavelength end the accuracy of such a system, thermal injury to tissue is significantly reduced or avoided.

The benefits of the combination of chromophores and laser may be further enhanced by partially setting the adhesive before ultimate application. This further reduces the final setting energy levels and has obvious clinical advantages.
There are a few major potential problems using lasers for bonding or activating adhesives or indeed using other energy sources. The time taken for setting or bonding is usually a few seconds. Exposure of just a few more seconds may damage the underlying tissue being treated or other surrounding tissue. The operator will usually see a faint glow when the appropriate setting temperature has been obtained. However, this is unreliable and indeed may be difficult in taxing surgical application or conditions. It is thus relatively easy for the operator to under-irradiate or to over-irradiate.

According to the present invention, in a composition of the type comprising a tissue adhesive and an additive adapted to absorb radiation and thereby promote setting of the adhesive, the additive substantially or completely ceases to absorb radiation when the adhesive is at least substantially set.

This invention uses "colour change" chromophores which will demonstrate to the operator that the suitable end point has been reached. The additive absorbs energy until the setting reaction is sufficiently complete, and the absorbance of the material that is used changes so as to significantly reduce or cease absorption of further energy. Thus not only has the end point been clarified, but also safety has been enhanced by reducing the possibility of further potentially damaging energy being absorbed.

The reaction may comprise the setting of an adhesive such as a composition which bonds to living or other tissue or other materials. For use with living tissue, the components may be modified to enhance structural heating and to encourage cellular ingrowth.

The additive may comprise a radiation-absorbing substance convertible on irradiation to give a substance having reduced absorption or which is non-absorbing. The additive may comprise a chromophore or group of chromophores which changes per se following irradiation or which reacts with another substance, e.g. a reducing agent such as EDTA or ascorbic acid, to change in the conditions brought about by irradiation.

The method may be used to affect setting of an adhesive or potential adhesive applied to living or other tissue, in which the adhesive is irradiated with radiation which is poorly absorbed by the tissue but well absorbed by the adhesive until it changes colour, which prevents undersirible effects as a consequence of over-irradiation.

The substance may be irradiated by laser light to effect the reaction, which may be in the IR, visible or UV region of the spectrum. The choice of wavelength and chromophore may be altered for different applications.

The Argon laser at 488 and 514 nm is suitable for many applications. Laser power output may be altered. 0.15W-0.3W being commonly used. Power may be reduced to 0.05W in thermally pre-treated adhesives, eg. pre-treatment in which the material is incubated to approximately 70% of setting temperature for 1-2 hours.

Irradiation times of some 10 seconds are commonly applied at 0.2W for small diameter anastomoses using the Argon laser, but may be varied. Preferably the chromophore's absorption should reduce to a level at which the same laser power could be applied as long again or twice as long again without giving rise to further thermal damage.

Laser spot sizes may also be altered for different application Frequently a spot size of 1mm diameter has been used. Power density may also be varied. Commonly 10W/cm² is used.

Thus the additive may comprise a chromophore and a reactant therefore adapted to effect the conversion - for example a suitable red/orange coloured dye having an absorption maximum in the 400 - 520 nm region for use with an Argon Laser.

Several examples of such a dye are available, such as fuchsin, phloxin, erythrosin and eosin. They may be presented dissolved in say Ringer's Solution and may have a concentration between 0.1 mol/dm³ and 0.05 mol/dm³, but may be varied. Other solutions may be used as illustrated in later examples.

The additive may comprise a reducing agent as a conversion effecting reagent. The reducing agent may comprise for example, ascorbic acid which may be present at 0.11mol/dm³ concentration or EDTA which may be present at 0.2 mol/dm³ concentration.

The invention also comprises a tissus bonding adhesive containing. the additives or similar appropriate additives described above. The adhesive may comprise albumin, which may be in solution, e.g. in Ringer's Solution between 10 and 40% w/w, or other appropriate solutions.

Adhesives containing additives and modifications according to the invention may be used in a variety of circumstances and be presented, applied and prepared in many ways. In addition to vascular anastomoses, gut, ureteric and urethral applications may be used. Tissues of all descriptions, including liver and kidney may be repaired both by application to lacerated areas as well as by closing haemorrhagic sites. They may also be applied directly or endoscopically to areas of resected or damaged lung parenchyma. They may also be applied to seal drainage tubes or to seal areas after tube removal.

The various adhesive recipes may be coated and/or pre-treated (as required) onto patches of material, synthetic or biological or onto prostheses, such as vascular prostheses, to aid joining such prostheses to the free ends or sides of veins or arteries - for example in bypass procedures. They may also be used for seeling or coating vascular grafts.

The invention is not limited to the additives, dyes, solvents, adhesives and reagents disclosed above, nor to the use of the same for the purpose referred to. Nevertheless, it is anticipated that a very important application of the method and the additives and adhesives will be in the area of human and veterinary surgery, and that a range of adhesives and additives suitable therefor will be developed for different purposes and applications in that area.

The following Example illustrates the invention.

### Example

0.4 g lyophilised powder bovine albumin (Sigma Chemicals A2153) was added gradually to, and dissolved in, with stirring, 1 cm³ Ringer's solution. 0.28 mg heparan sulphate sodium salt (Fluka Biochemicals 51541) was added, and the solution was stirred slowly to ensure that the heparan sulphate had dissolved. 13.59 mg Eosin Y (Aldrich Chemicals 11,983-0) was then added to the 1 cm³ bovine albumin/heparan sulphate mixture, to give a 0.0196 mol/dm³ concentration of chromophore (Eosin Y). The solution was split into two 0.5 cm³ fractions.

The first fraction was used as a control. To the second 0.5 cm³ fraction was added 0.2 cm³ of a chondroitin sulphate A solution. This solution was prepared by gradual addition of 0.1 g chondroitin sulphate A sodium salt (Sigma Chemicals C 9819) to 1 cm³ Ringer's solution, with stirring.

Ethylenediaminetetraacetic acid (EDTA) (the chemical responsible for dye fading) as the disodium salt dihydrate (Aldrich Chemicals 25,235-2; 772.98 mg) was dissolved in 10 cm³ Ringer's solution. 0.1 cm³ of this solution, containing 0.20 mol/dm³ EDTA, was then added to 0.7 cm³ of the bovine albumin/chondroitin sulphate A solution. The resultant concentration of EDTA was 0.025 mol/dm³.

The Control and the Example solutions were used in end-to-end anastomoses of porcine splenic arteries, using a laser power of 0.2 watts and spot size of 1 mm. Burst pressures for 8 and 9 experiments respectively were as follows:
Control: 138.1, 250, 446.5, 219.8, 421.8, 272.7, 85.4, 89.7 mmHg

### Example: 495.8, 259.2, 308.6, 482.4, 403.9, 366.9, 317.6, 296.3, 291.8 mmHg

Although there was no appreciable time difference, in performing the anastomoses in either group, the bursting pressures were significantly higher for the Example. Histological examination of both groups demonstrated significant thermal injury in the group without fading chromophore (Control) and no injury or minimal thermal damage in the chromophore fading group (Example).

## Claims

1. A composition comprising a tissue adhesive and an additive adapted to absorb radiation and which on absorption of radiation promotes setting of the adhesive, characterised in that the additive substantially or completely ceases to absorb radiation when the adhesive is at least substantially set.

2. A composition according to claim 1, wherein the additive comprises a chromophore which changes per se on irradiation or which reacts with another substance to change in the conditions brought about by irradiation.

3. A composition according to claim 1 or claim 2, wherein the additive comprises a red/orange-coloured dye.

4. An composition according to claim 3, wherein the dye has an absorption maximum in the 400-520 nm region.

5. A composition according to claim 3 or claim 4, wherein the dye comprises basic fuchsin.

6. A composition according to claim 3 or claim 4,wherein the dye comprises philoxim.

7. A composition according to claim 3 or claim 4, wherein the dye comprises erythrosin.

8. A composition according to claim 3 or claim 4, wherein the dye comprises eosin.

9. A composition according to any preceding claim, wherein the additive further comprises a reducing agent.

10. A composition according to claim 9, wherein the reducing agent is ascorbic acid.

11. A composition according to claim 9, wherein the reducing agent is EDTA.

12. A composition according to any preceding claim, wherein the tissue adhesive further comprises albumin.

## Patentansprüche

1. Zusammensetzung, umfassend einen Gewebekleber und ein Additiv, welches zur Absorption von Strahlung geeignet ist und welches nach Absorption von Strahlung die Aushärtung des Klebers fördert, dadurch gekennzeichnet, daß das Additiv die Absorption von Strahlung im wesentlichen oder völlig einstellt, wenn der Kleber mindestens im wesentlichen ausgehärtet ist.

2. Zusammensetzung nach Anspruch 1, worin das Additiv einen Chromophor umfaßt, welcher sich bei Bestrahlung von selbst umwandelt oder welcher mit einer anderen Substanz reagiert, um sich unter den von der Bestrahlung erzeugten Bedingungen umzuwandeln.

3. Zusammensetzung nach Anspruch 1 oder Anspruch 2, worin das Additiv einen Farbstoff von roter/oranger Farbe umfaßt.

4. Zusammensetzung nach Anspruch 3, worin der Farbstoff ein Absorptionsmaximum im Bereich von 400-520 nm aufweist.

5. Zusammensetzung nach Anspruch 3 oder Anspruch 4, worin der Farbstoff basisches Fuchsin umfaßt.

6. Zusammensetzung nach Anspruch 3 oder Anspruch 4, worin der Farbstoff Philoxim umfaßt.

7. Zusammensetzung nach Anspruch 3 oder Anspruch 4, worin der Farbstoff Erythrosin umfaßt.

8. Zusammensetzung nach Anspruch 3 oder Anspruch 4, worin der Farbstoff Eosin umfaßt.

9. Zusammensetzung nach irgendeinem der vorhergehenden Ansprüche, worin das Additiv ferner ein Reduktionsmittel umfaßt.

10. Zusammensetzung nach Anspruch 9, worin das Reduktionsmittel Ascorbinsäure ist.

11. Zusammensetzung nach Anspruch 9, worin das Reduktionsmittel EDTA ist.

12. Zusammensetzung nach irgendeinem der vorhergehenden Ansprüche, worin der Gewebekleber ferner Albumin umfaßt.

## Revendications

1. Composition comprenant un adhésif pour tissu et un additif prévu pour absorber un rayonnement et qui, lors de l'absorption d'un rayonnement, facilite la prise de l'adhésif, caractérisée en ce que l'additif cesse essentiellement ou complètement d'absorber le rayonnement lorsque l'adhésif est au moins substantiellement pris.

2. Composition selon la revendication 1, dans laquelle l'additif comprend un chromophore qui change en soi lors d'une irradiation, ou qui réagit avec une autre substance pour changer dans les conditions provoquées par l'irradiation.

3. Composition selon la revendication 1 ou la revendication 2, dans laquelle l'additif comprend un colorant de couleur rouge/orange.

4. Composition selon la revendication 3, dans laquelle le colorant présente une absorption maximum dans la région de 400-520 nm.

5. Composition selon la revendication 3 ou la revendication 4, dans laquelle le colorant comprend de la fuchsine basique.

6. Composition selon la revendication 3 ou la revendication 4, dans laquelle le colorant comprend de la phloxine.

7. Composition selon la revendication 3 ou la revendication 4, dans laquelle le colorant comprend de l'érythrosine.

8. Composition selon la revendication 3 ou la revendication 4, dans laquelle le colorant comprend de l'éosine.

9. Composition selon l'une quelconque des revendications précédentes, dans laquelle l'additif comprend de plus un agent réducteur.

10. Composition selon la revendication 9, dans laquelle l'agent réducteur est l'acide ascorbique.

11. Composition selon la revendication 9, dans laquelle l'agent réducteur est l'EDTA.

12. Composition selon l'une quelconque des revendications précédentes, dans laquelle l'adhésif pour tissu comprend de plus de l'albumine.
